Europäisches Patentamt

(19) European Patent Office

Office européen des brevets

(11) **EP 1 268 380 B1**

(12) **EUROPÄISCHE PATENTSCHRIFT**

(45) Veröffentlichungstag und Bekanntmachung des Hinweises auf die Patenterteilung:
**17.09.2003 Patentblatt 2003/38**

(21) Anmeldenummer: **01921359.4**

(22) Anmeldetag: **29.03.2001**

(51) Int Cl.⁷: **C07C 41/08**, C07C 43/16

(86) Internationale Anmeldenummer:
**PCT/EP01/03588**

(87) Internationale Veröffentlichungsnummer:
**WO 01/077051 (18.10.2001 Gazette 2001/42)**

(54) **VERFAHREN ZUR HERSTELLUNG VON ALKENYLETHERN**

METHOD FOR PRODUCING ALKENYL ETHERS

PROCEDE DE PRODUCTION D'ETHERS D'ALCENYLE

(84) Benannte Vertragsstaaten:
**AT BE CH CY DE DK ES FI FR GB GR IE IT LI LU MC NL PT SE TR**

(30) Priorität: **06.04.2000 DE 10017222**

(43) Veröffentlichungstag der Anmeldung:
**02.01.2003 Patentblatt 2003/01**

(73) Patentinhaber: **BASF AKTIENGESELLSCHAFT**
**67056 Ludwigshafen (DE)**

(72) Erfinder:
• **BÖTTCHER, Arnd**
**67227 Frankenthal (DE)**
• **PINKOS, Rolf**
**67089 Bad Dürkheim (DE)**
• **LORENZ, Rudolf Erich**
**67069 Ludwigshafen (DE)**

(56) Entgegenhaltungen:
EP-A- 0 367 087          WO-A-91/05756
DD-A- 298 775            DD-A- 298 776
DE-A- 1 812 602

• **E. D. HOLLY: "Vinylation rates of primary, secondary, and tertiary alcohols" JOURNAL OF ORGANIC CHEMISTRY, Bd. 24, November 1959 (1959-11), Seiten 1752-1755, XP002170553 EASTON US in der Anmeldung erwähnt**

EP 1 268 380 B1

**Beschreibung**

Die vorliegende Erfindung betrifft ein verbessertes Verfahren zur Herstellung von Alkenyl-ethern durch Umsetzung der entsprechenden Alkohole mit Acetylenen in der Flüssigphase in Gegenwart basischer Alkalimetallverbindungen und eines Cokatalysators.

Alkenyl-ether finden unter anderem Verwendung als Monomerbausteine in Polymeren beziehungsweise Copolymeren, in Beschichtungen, Klebstoffen, Druckfarben sowie in strahlungshärtenden Lacken. Weitere Anwendungsgebiete sind die Herstellung von Zwischenprodukten, Geruchs- und Geschmacksstoffen sowie pharmazeutischen Produkten.

Die technische Herstellung von Vinylethern erfolgt in der Regel durch Umsetzung der entsprechenden Alkohole mit Ethin in Gegenwart basischer Katalysatoren (siehe Ullmann's Encyclopedia of Industrial Chemistry, 6th edition, 1999 Electronic Release, Chapter "Vinyl Ethers - Production"). Die Vinylierung kann dabei sowohl in der Flüssigphase als auch in der Gasphase durchgeführt werden. Bei der Vinylierung in der Gasphase werden basische Heterogenkatalysatoren, wie beispielsweise KOH auf Aktivkohle oder MgO oder CaO eingesetzt. In der Flüssigphase wird die stark exotherme Reaktion im allgemeinen in Gegenwart von Alkalimetall-alkoxid-Katalysatoren, speziell in Gegenwart der Kalium-alkoholate der eingesetzen Alkohole, bei Temperaturen von 120 bis 180°C durchgeführt. Bei der Vinylierung von primären und sekundären aliphatischen Alkoholen läuft die Reaktion im allgemeinen bereitwillig ab. Tertiäre aliphatische Alkohole lassen sich aufgrund ihrer geringeren Reaktivität in der Regel jedoch nur langsam und unvollständig vinylieren. Vor allem bei der Vinylierung tertiärer Alkohole werden im allgemeinen beträchtliche Mengen unerwünschter, teilweise nicht-flüchtiger Nebenprodukte gebildet. Gleiches gilt prinzipiell auch für den Einsatz von phenolischen Ausgangsstoffen.

Die starke Abnahme der Reaktivität von primären über sekundäre zu tertiären Alkohlen ist für die Vinylierung in der Gasphase beschrieben in V.A. Sims and J.F. Vitcha, Ind. Eng. Chem. Prod. Res. Dev., Vol. 2, No. 4, 1963, Seite 293-296, und für die Vinylierung in der Flüssigphase in E.D. Holly, J. Org. Chem. Vol. 24, 1959, Seite 1752-1755. Wie bei B. Trofimov, Z. Chem. Bd. 26, 1986, Heft 2, Seite 41-49 beschrieben ist, ist auch in Gegenwart superbasischer Medien die Ausbeute bei der Vinylierung tertiärer Alkohole gering.

In zahlreichen Veröffentlichungen ist eine Verdünnung der Reaktionslösung durch Einsatz verschiedener Lösungsmittel beschrieben. Der Lösungsmittelzusatz ermöglicht eine Vinylierung niedermolekularer Alkohole unter einem milden Druck, vielfach Atmosphärendruck. Als geeignete Lösungsmittel sind beispielsweise aus SU 481 589 und SU 1 504 969 höhersiedende Nebenprodukte, welche bei der Vinylierung parallel entstehen, bekannt. GB 717 051 lehrt als geeignete Lösungsmittel den Einsatz höhermolekularer Vinylether, welche beispielsweise in einer vorgelagerten Vinylierungsreaktion aus einem höhermolekularen Alkohol synthetisiert werden können. Mono-, Oligo- und Polyglykole des Ethylen-, Propylenoder Butylenoxids sowie deren Ether als geeignete Lösungsmittel sind in JP 04 198 144 A2 und JP 04 095 040 A2 beschrieben.

DD-A 298 775 und DD-A 298 776 offenbaren die Synthese von Alkylvinylethern unter dem Einsatz eines Lösungsmittels, beispielsweise einem aliphatischen, cycloaliphatischen oder aromatischen Kohlenwasserstoff oder einem Ether, und in Gegenwart eines Mono-, Oligo- oder Polyglykols des Ethylen-, Propylen- oder Butylenoxids oder eines 18-Krone-6- oder Dibenzo-18-krone-6-Kronenethers als Cokatalysator.

Gemäß der Lehre der Offenlegungsschrift DE-A 1 812 602 wird durch den Einsatz von Mono-, Oligo- und Polyglykolen des Ethylen-, Propylen- oder Butylenoxids sowie deren Ether als Lösungsmittel für die Herstellung von Aryl-vinyl-ethern eine kontinuierliche Durchführung der Reaktion ermöglicht, da die Bildung von Ablagerungen unterbunden und die Ausbeute an Wertprodukt erhöht wird.

In WO 91/05756 ist der Einsatz von Tetraethylenglykoldimethylether als Lösungsmittel bei der Vinylierung und Epoxyvinylierung von 1,1,1-Tris(hydroxymethyl)-ethan beschrieben.

Die in den genannten Veröffentlichungen offenbarten Mengen an Lösungsmitteln betragen vielfach mehr als die Hälfte der gesamten Masse an eingesetzem Alkohol. Erfindungsgemäß wurde erkannt, daß aufgrund des benötigten Reaktionsvolumens nur eine unbefriedigend niedrige Raum/Zeit-Ausbeute erreicht wird. Des weiteren wurde erfindungsgemäß erkannt, daß die hohen Gehalte an Lösungsmitteln sowohl bei der Synthese als auch bei der anschließenden destillativen Aufarbeitung einen hohen Energieverbrauch nach sich ziehen (z.B. Heizenergie, Kühlenergie). Erfindungsgemäß wurde auch erkannt, daß bei Einsatz der Mono-, Oligo- und Polyglykole aufgrund der noch reaktiven Hydroxygruppen ein erheblicher Teil des zugegebenen Acetylens für deren Vinylierung verbraucht wird und somit der eigentlichen Vinylierungsreaktion nicht mehr zur Verfügung steht. Bei den eingesetzten Lösungsmitteln handelt es sich um größere Mengen vielfach teurer Einsatzstoffe, welche in der Regel nicht wiedergewonnen werden können.

Es bestand daher die Aufgabe, ein Verfahren zur Herstellung von Alkenyl-ethern zu entwickeln, welches die aufgeführten Nachteile nicht besitzt und die Darstellung der Alkenyl-ether mit einer hohen Raum/Zeit-Ausbeute in einfacher Art und Weise ermöglicht. Insbesondere sollte dabei die Bildung von Nebenprodukten bei gleichzeitig konzentrierter Fahrweise erheblich zurückgedrängt werden, so daß höchstens nur eine geringe Menge an nicht-flüchtigen Rückstand gebildet wird und ein Zähflüssigwerden oder eine Verfestigung des Reaktionsaustrags unterbunden wird.

Demgemäß wurde ein Verfahren zur Herstellung von Alkenyl-ethern durch Umsetzung der entsprechenden Alkohole oder Phenole mit Acetylenen in der Flüssigphase in Gegenwart basischer Alkalimetallverbindungen und eines Cokatalysators gefunden, das dadurch gekennzeichnet ist, daß man als Cokatalysator Verbindungen der allgemeinen Formeln (Ia) und/oder (Ib)

$$\text{(Ia):} \quad R^1 O\text{-}(CH_2CH_2CH_2CH_2O)_{n}\text{-}H$$

$$\text{(Ib):} \quad R^1 O\text{-}(CH_2CH_2CH_2CH_2O)_{n}\text{-}R^2,$$

worin $R^1$, $R^2$ unabhängig voneinander $C_1$- bis $C_6$-Alkyl oder $C_2$- bis $C_6$-Alkenyl, oder $R^1$ und $R^2$ gemeinsam eine Butenyl-Einheit, bedeuten und n 1, 2, 3, 4 oder 5 entspricht, einsetzt.

Nach dem erfindungsgemäßen Verfahren lassen sich Alkenyl-ether aus den entsprechenden Alkohole oder Phenolen und Acetylenen in Gegenwart basischer Alkalimetallverbindungen und eines preiswerten Cokatalysators, welcher einfach aus dem Reaktionsgemisch wieder abzutrennen ist, in hoher Selektivität und hoher Ausbeute gewinnen.

Wesentlich beim erfindungsgemäßen Verfahren ist die Gegenwart eines Cokatalysators (Ia) und/oder (Ib)

$$\text{(Ia):} \quad R^1 O\text{-}(CH_2CH_2CH_2CH_2O)_{n}\text{-}H$$

$$\text{(Ib):} \quad R^1 O\text{-}(CH_2CH_2CH_2CH_2O)_{n}\text{-}R^2,$$

worin $R^1$, $R^2$ unabhängig voneinander unverzweigtes oder verzweigtes $C_1$- bis $C_6$-Alkyl, beispielsweise Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;

oder unverzweigtes oder verzweigtes $C_2$- bis $C_6$-Alkenyl mit einer Doppelbindung in einer beliebigen Position, beispielsweise Ethenyl (Vinyl), 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Hexenyl, 2-Hexenyl, 3-Hexenyl, 4-Hexenyl und 5-Hexenyl;

oder $R^1$ und $R^2$ gemeinsam eine Butenyl-Einheit, konkret $CH_2CH_2CH_2CH_2$, bedeuten und n 1, 2, 3, 4 oder 5 entspricht, oder Gemische davon.

Als Beispiele der im erfindungsgemäßen Verfahren einzusetzenden Cokatalysatoren (Ia) und/oder (Ib) seien genannt 4-Methoxy-1-butanol, 4-Ethoxy-1-butanol, 4-Propoxy-1-butanol, 4-Butoxy-1-butanol, 1,4-Dimethoxy-butan, 1,4-Diethoxy-butan, 1,4-Dipropoxybutan, 1,4-Dibutoxy-butan, 1-Ethoxy-4-methoxy-butan, 1-Propoxy-4-methoxy-butan, 1-Butoxy-4-methoxy-butan, 1-Propoxy-4-ethoxybutan, 1-Butoxy-4-ethoxy-butan, 1-Butoxy-4-propoxy-butan, 4-Vinyloxy-1-butanol, 4-(Isopropenyloxy)-1-butanol, 4-Propenyloxy-1-butanol, 1,4-Divinyloxy-butan, 1,4-Bis(Isopropenyloxy)-butan, 1,4-Bis(propenyloxy)-butan, 1-Vinyloxy-4-methoxy-butan, 1-Vinyloxy-4-ethoxy-butan, 1-Vinyloxy-4-propoxy-butan, 1-(Isopropenyloxy)-4-propoxy-butan, 1-(Propenyloxy)-4-propoxy-butan, 4-(4'-Methoxy-1'-butoxy)-1-butanol, 4-(4'-Ethoxy-1'-butoxy)-1-butanol, 4-(4'-Vinyloxy-1'-butoxy)-1-butanol, Bis-(4-methoxy-1-butyl)-ether, Bis-(4-ethoxy-1-butyl)-ether, Bis-(4-vinyloxy-1-butyl)-ether, 10-Krone-2, 15-Krone-3 und 20-Krone-4.

Bevorzugt eingesetzt werden Cokatalysatoren der Formeln (Ia) und/oder (Ib), worin $R^1$, $R^2$ unabhängig voneinander Ethyl oder Vinyl bedeuten, beispielsweise 4-Ethoxy-1-butanol, 1,4-Diethoxy-butan, 4-Vinyloxy-1-butanol, 1,4-Divinyloxy-butan, 1-Vinyloxy-4-ethoxybutan, 4-(4'-Ethoxy-1'-butoxy)-1-butanol, 4-(4'-Vinyloxy1'-butoxy)-1-butanol, Bis-(4-ethoxy-1-butyl)-ether und Bis-(4-vinyloxy-1-butyl)-ether oder Gemische davon.

Besonders bevorzuge eingesetzt werden 4-Ethoxy-1-butanol, 1,4-Diethoxy-butan, 4-Vinyloxy-1-butanol, 1,4-Divinyloxy-butan, 1-Vinyloxy-4-ethoxy-butan oder Gemische davon. Ganz besonders bevorzugt eingesetzt werden 1,4-Diethoxy-butan, 1,4-Divinyloxy-butan oder Gemische davon.

Die im erfindungsgemäßen Verfahren einzusetzenden Cokatalysatoren können durch die folgenden Synthesen erhalten werden:

a) 4-Alkenyloxy-1-butanole und 1,4-Dialkenyloxy-butane werden durch Umsetzung von 1,4-Butandiol mit Acetylenen in Gegenwart eines basischen Katalysators gebildet und destillativ getrennt. So können 4-Vinyloxy-1-butanol und 1,4-Divinyloxy-butan beispielsweise durch Umsetzung von 1,4-Butandiol mit Ethin und destillativer Aufarbeitung gewonnen werden.

b) 4-Alkoxy-1-butanole und 1,4-Dialkoxy-butane werden durch katalytische Hydrierung der nach Beschreibung (a) erhaltenen 4-Alkenyloxy-1-butanole und 1,4-Dialkenyloxy-butane hergestellt. Geeignete Hydrierkatalysatoren sind dem Fachmann bekannt. Es können beispielsweise eingesetzt werden Edelmetall-Pulver, -Mohr oder -Schwarz, geträgerte Hydriermetalle, wie z.B. Edelmetalle, Nickel oder Kupfer auf Aktivkohle oder oxidischen Trägermaterialien oder Raney-Katalysatoren, wie etwa Raney-Nickel. 1,4-Diethoxy-butan kann somit durch Hydrierung von 1,4-Divinyloxy-butan erhalten werden.

Alternativ sind die 4-Alkoxy-1-butanole und 1,4-Dialkoxy-butane auch durch Veretherung von 1,4-Butandiol mit den entsprechenden Alkanolen nach den, dem Fachmann bekannten Veretherungsvorschriften, erhältlich.

c) 1-Alkenyloxy-4-alkoxy-butane werden durch Umsetzung der nach Beschreibung (b) erhaltenen 4-Alkoxy-1-butanole mit Acetylenen entsprechend der Beschreibung (a) erhalten.

d) Alkoxy- und Alkenyloxy-Derivate von Dibutylenglycol und Tributylenglycol können durch intermolekulare Veretherung von 1,4-Butandiol und anschließende Derivatisierung entsprechend der Beschreibungen (a) bis (c) erhalten werden.

e) 1,4-Butandiol-Kronenether können durch intermolekulare Veretherung von 1,4-Butandiol erhalten werden.

Der erfindungsgemäß zu verwendende Cokatalysator wird vorteilhaft in einer Menge von 0,1 bis 10 Gew.-%, bezogen auf den eingesetzten Alkohol oder das eingesetzte Phenol, eingesetzt. Besonders bevorzugt ist eine Menge von 0,5 bis 5 Gew.-%.

Alkohole, welche beim erfindungsgemäßen Verfahren als Ausgangsstoff eingesetzt werden, sind aus der Gruppe Methanol, Ethanol, 1-Propanol, 2-Propanol, 1-Butanol, 2-Butanol (sek. Butanol), 2-Methyl-1-propanol (Isobutanol), 1-Methyl-2-propanol (tert. Butanol), 1-Pentanol, 2-Pentanol, 3-Pentanol, 2-Methyl-1-butanol, 3-Methyl-1-butanol (Isoamylalkohol), 2,2-Dimethyl-1-propanol, 2-Methyl-2-butanol, 3-Methyl-2-butanol, 1-Hexanol, 2-Hexanol, 3-Hexanol, 3,3-Dimethyl-3-butanol, 4-Methyl-2-pentanol, 2-Ethyl-1-butanol, cis-3-Hexen-1-ol, 5-Hexen-1-ol, 1-Heptanol, 2-Heptanol, 3-Heptanol, 2,4-Dimethyl-3-pentanol, 1-Octanol, 2-Octanol, 3-Octanol, 2-Ethyl-1-hexanol, 2,4,4-Trimethyl-1-pentanol, 1-Nonanol, 2-Nonanol, 3-Nonanol, 4-Nonanol, 5-Nonanol, 1-Decanol, 2,2-Dimethyl-1-octanol, 1-Dodecanol, 1-Tetradecanol (Myristylalkohol), 1-Hexadecanol (Cetylalkohol), 1-Octadecanol (Stearylalkohol), cis-9-Octadecen-1-ol (Oleylalkohol), cis,cis-9,12-Octadecadien-1-ol, cis,cis,cis-9,12,15-Octadecatrien-1-ol, 1-Eicosanol (Arachylalkohol), 1-Docosanol (Behenylalkohol),

Cyclopropanol, Cyclopropylmethanol, Cyclopropylethanol, Cyclobutanol, Cyclobutylmethanol, Cyclobutylethanol, Cyclopentanol, Cyclopentylmethanol, Cyclopentylethanol, 1-Methyl-cyclopentanol, 2-Methyl-cyclopentanol, 3-Methyl-cyclopentanol, Cyclohexanol, Cyclohexylmethanol, Cyclohexylethanol, 1-Methyl-cyclohexanol, 2-Methyl-cyclohexanol, 3-Methyl-cyclohexanol, 4-Methyl-cyclohexanol, Cycloheptanol, Cyclooctanol, Cyclodecanol, Benzylalkohol, Hydroxydiphenylmethan, 1-Phenyl-ethanol, 2-Phenyl-ethanol, 2,2-Diphenyl-ethanol, 2,2,2-Triphenylethanol,

1,2-Ethandiol, 1,2-Propandiol, 1,3-Propandiol, 1,2-Butandiol, 1,5-Pentandiol, 1,6-Hexandiol, Diethylenglykol, Triethylenglykol, Tetraethylenglykol, 1,2,3-Propantriol (Glycerin) und 2-Methyl-1,2,3-propantriol ausgewählt.

Als Phenole, welche beim erfindungsgemäßen Verfahren als Ausgangsstoff eingesetzt werden, sind all jene Verbindungen mit 1 bis 12 Kohlenstoffatomen und deren Derivate zu verstehen, welche mindestens eine, an einem aromatischen Kohlenstoff gebundene Hydroxygruppe aufweisen.

Aus der Reihe der Phenole seien beispielsweise genannt Phenol, 2-Methylphenol (o-Kresol), 3-Methylphenol (m-Kresol), 4-Methylphenol (p-Kresol), 2-Ethylphenol (o-Kresol), 3-Ethylphenol (m-Kresol), 4-Ethylphenol (p-Kresol), 2,3-Dimethylphenol, 2,4-Dimethylphenol, 2,5-Dimethylphenol, 2,6-Dimethylphenol, 3,4-Dimethylphenol, 3,5-Dimethylphenol, 1-Naphthol, 2-Naphthol.

Es können natürlich auch Verbindungen eingesetzt werden, welche sowohl alkoholische als auch phenolische Hydroxygruppen besitzen, wie beispielsweise 2-(4'-Hydroxyphenyl)-ethanol.

Bevorzugt beim erfindungsgemäßen Verfahren ist der Einsatz von aliphatischen Alkoholen. Besonders bevorzugt sind die nichtcyclischen, gesättigten, aliphatischen Alkohole mit 1 bis 6 Kohlenstoffatomen, insbesondere mit 3 bis 5 Kohlenstoffatomen, wie beispielsweise 1-Methyl-2-propanol (tert. Butanol) und 3-Methyl-1-butanol (Isoamylalkohol).

Als Acetylene werden beim erfindungsgemäßen Verfahren bevorzugt unverzweigte und verzweigte Alkine mit 2 bis 6 C-Atomen und einer endständigen Dreifachbindung eingesetzt, wie beispielsweise Ethin, Propin, 1-Butin, 1-Pentin, 1-Hexin. Besonders bevorzugt ist der Einsatz von Ethin und Propin, insbesondere von Ethin.

Als basische Alkalimetallverbindungen, welche auch als Katalysator bezeichnet werden, können beim erfindungsgemäßen Verfahren Alkoholate und/oder Phenolate von Lithium, Natrium, Kalium, Rubidium und/oder Cäsium sowie deren Gemische eingesetzt werden. Bevorzugt sind die Alkalimetallverbindungen des Natriums und des Kaliums.

Die basischen Alkalimetallverbindungen werden im allgemeinen in einer Menge von 0,02 bis 12%, bevorzugt von 0,05 bis 10%, der Molmenge des eingesetzten Alkohols oder Phenols eingesetzt.

Es ist im allgemeinen vorteilhaft, die Alkalimetallalkoholate oder -phenolate der in der Alkenylierung eingesetzten Alkohole oder Phenole zu verwenden, da dadurch keine weiteren Fremdstoffe in das Reaktionssystem eingebracht werden, welche entweder in einem vorgelagertem Schritt abzutrennen wären oder die Ausbeute an Wertprodukt verringern würden. Die entsprechenden Alkalimetallverbindungen können bei Bedarf nach den bekannten Verfahren hergestellt werden. Als geeignete Verfahren seien beispielsweise die Umsetzung aus den entsprechenden Alkoholen oder Phenolen mit (i) elementaren Alkalimetallen, (ii) mit Hydroxiden und Entfernung des gebildeten Reaktionswassers und (iii) mit sogenannten Fremdalkoholen oder -phenolen und Entfernung der gebildeten Fremalkohole oder -phenole, genannt.

In bestimmten Fällen ist es jedoch vorteilhaft, bewußt sogenannte Fremdalkoholate oder -phenolate zuzugeben, beispielsweise in den Fällen, in denen die entsprechenden Alkoholate oder Phenolate der eingesetzten Alkohole oder Phenole nicht verfügbar sind und eine vorgelagerte, separate Synthese nicht in Frage kommt. Bevorzugt sind die niedermolekularen Verbindungen einzusetzen, da diese sich in ihren Siedepunkten deutlich von den eingesetzten Alkoholen oder Phenolen unterscheiden und eine leichte destillative Abtrennung ermöglichen. Von den niedermolekularen Alkoholaten bevorzugt sind die Methanolate, Ethanolate, Propanolate und 2-Propanolate des Natriums und Kaliums. Die zugegebenen Fremdalkoholate oder -phenolate werden bei dieser Variante ebenfalls alkenyliert und sind anschließend von dem Wertprodukt abzutrennen. Alternativ können die bei der Umsetzung zwischen den Fremdalkoholaten und -phenolaten mit den Alkoholen oder Phenolen gebildeten Fremdalkohole oder -phenole auch vor der Alkenylierung abgetrennt werden, beispielsweise destillativ.

Werden als Ausgangsstoffe für die basischen Alkalimetallverbindungen Alkalimetallhydroxide eingesetzt, so ist vor der Alkenylierung das gebildete Reaktionswasser zu entfernen. Geeignet Methoden sind beispielsweise die Abdestillation des Wassers, die Adsorption an einem geeigneten Trocknungsmittel oder die Ausschleusung durch eine geeignete Membran. Es ist vorteilhaft, einen Restgehalt an Wasser von kleiner 1 Gew.-%, bevorzugt kleiner 0,5 Gew.-%, besonders bevorzugt kleiner 0,2 Gew.-%, bezogen auf die Gesamtflüssigkeitsmenge, einzustellen. Die entsprechenden Verfahren sind dem Fachmann bekannt. Bei dieser Variante ist der Einsatz von Natrium- und/oder Kaliumhydroxid bevorzugt.

Das erfindungsgemäße Verfahren kann in Gegenwart eines geeigneten Lösungsmittels durchgeführt werden. Der Einsatz eines Lösungsmittels ist beispielsweise bei zähflüssigen oder gar festen Einsatzstoffen vorteilhaft oder sogar für den Erfolg des Verfahrens erforderlich. Geeignete Lösungsmittel zeichnen sich dadurch aus, daß sie unter den Reaktionsbedingungen inert sind und sich problemlos von den Wertprodukten, beispielsweise destillativ, abtrennen lassen. Beispiele geeigneter Lösungsmittel sind N-Methylpyrrolidon, Tetrahydrofuran oder Dialkylether von Glykolen, Di-, Oligo- oder Polyglykolen. Bei flüssigen Einsatzstoffen und/oder flüssigen Reaktionsprodukten ist die Ausübung des erfindungsgemäßen Verfahrens ohne Zusatz eines Lösungsmittels bevorzugt.

Die Reihenfolge der Zugaben des Alkohols oder Phenols und der basischen Alkalimetallverbindung sowie des Cokatalysators und gegebenenfalls eines Lösungsmittels sind für den Erfolg des Verfahrens unwesentlich. Wichtig ist, daß der Wassergehalt der Reaktionslösung vor der Alkenylierung unterhalb der oben angegebenen Werte liegt. Das Acetylen wird im allgemeinen entsprechend dem Fortschreiten der Reaktion zudosiert.

Das erfindungsgemäße Verfahren kann diskontinuierlich, halbkontinuierlich oder kontinuierlich durchgeführt werden. Bevorzugt sind die halbkontinuierliche oder kontinuierliche Variante. Beim halbkontinuierlichen Verfahren wird die Alkohol oder Phenol, die basische Alkalimetallverbindung, den Cokatalysator und gegebenenfalls ein Lösungsmittel enthaltende Lösung vorgelegt und das Acetylen, entsprechend dem Reaktionsverlauf, zudosiert. Die Produktlösung wird normalerweise erst nach Beendigung der Reaktion entnommen. Beim kontinuierlichen Verfahren werden die Alkohol oder Phenol, die basische Alkalimetallverbindung, den Cokatalysator und gegebenenfalls ein Lösungsmittel enthaltende Lösung und das Acetylen kontinuierlich zugeführt und die entstandene Lösung der Reaktionsprodukte kontinuierlich abgeführt.

Die Alkenylierung wird im allgemeinen bei einer Temperatur von 100 bis 200°C, bevorzugt von 130 und 180°C, besonders bevorzugt von 140 bis 170°C durchgeführt. Sie wird im allgemeinen bei einem Acetylendruck von kleiner 5 MPa (50 bar), bevorzugt von kleiner 3 MPa (30 bar), ganz besonders bevorzugt von kleiner 2,4 MPa (24 bar) durchgeführt. Der Gesamtdruck des Systems kann jedoch deutlich höher liegen, da die überstehende Gasatmosphäre beispielsweise noch Inertgase, wie Stickstoff oder Edelgase, welche durch gezieltes Aufpressen eingebracht werden können, enthalten kann. So ist ein Gesamtdruck im System von beispielsweise 20 MPa abs (200 bar abs) ohne weiteres möglich. Werden höhermolekulare Acetylene eingesetzt, so ist der sich einstellende Acetylendruck sehr niedrig und kann beispielsweise deutlich unterhalb von 0,1 MPa (1 bar) liegen. Bei den niedermolekularen Aycetylenen, wie etwa Ethin, Propin und 1-Butin, wird im allgemeinen ein Acetylendruck von größer 0,1 MPa (1 bar) eingestellt. Dadurch wird eine wirtschaftliche Raum/Zeit-Ausbeute erreicht. Wird bei der Alkenylierung Ethin als Acetylen eingesetzt, so wird sie bevorzugt bei einem Acetylendruck (Ethindruck) von 0,5 bis 3,0 MPa (5 bis 30 bar), besonders bevorzugt von 0,8 bis 2,4 MPa (8 bis 24 bar) und ganz besonders bevorzugt von 1,6 bis 2,0 MPa (16 bis 20 bar) durchgeführt.

Als Reaktoren für die Alkenylierung können prinzipiell die in der einschlägigen Fachliteratur beschriebenen Apparate für gas/flüssig Umsetzungen eingesetzt werden. Zur Erzielung einer hohen Raum/Zeit-Ausbeute ist eine intensive Durchmischung zwischen der Alkohol oder Phenol, der basische Alkalimetallverbindung, den Cokatalysator und gegebenenfalls ein Lösungsmittel enthaltende Lösung und dem Acetylen wichtig. Als nicht-einschränkende Beispiele seien genannt Rührkessel, Rührkesselkaskade, Strömungsrohre (bevorzugt mit Einbauten), Blasensäulen und Schlaufenreaktoren.

Der Reaktionsaustrag wird nach bekannten Methoden aufgearbeitet. Bevorzugt ist eine Destillation in mehrere Fraktionen. Die Destillationen werden bevorzugt bei einem Druck kleiner 0,1 MPa abs (1 bar abs) durchgeführt. Besonders bevorzugt werden neben dem Alkenyl-ether auch die Cokatalysatoren als Fraktion gewonnen. Je nach Wahl der erfindungsgemäß zu verwendenden Cokatalysatoren werden sie in einer niedrigersiedenden oder höhersiedenden Fraktion vor oder nach dem Alkenyl-ether abgetrennt. Im folgenden seien, ohne limitierend zu wirken, verschiedene Fraktionen genannt: Cokatalysator (vor oder nach Alkenyl-ether), Alkenyl-ether, nicht-umgesetzter Alkohol oder nicht-umgesetztes Phenol, diverse Zwischensieder, Leichtsieder und Schwersieder. Je nach Intention können diese als Rohfraktion oder in hoher Reinheit gewonnen werden. Es ist auch möglich, einige Fraktionen zusammenzufassen. Die Destillation kann diskontinuierlich, halbkontinuierlich oder kontinuierlich durchgeführt werden. Zudem kann sie in einer Kolonne, gegebenenfalls mit Seitenabzügen, als auch in mehreren, hintereinandergeschalteten Kolonnen durchgeführt werden. Geeignete Verfahren sind dem Fachmann bekannt. Der Alkenylether kann durch das erfindungsgemäße Verfahren, wie beschrieben, in einfacher Art und Weise mit einem Reinheitsgrad von über 99% erhalten werden.

Es ist prinzipiell beim erfindungsgemäßen Verfahren möglich, den gegebenenfalls abgetrennten, nicht-umgesetzten Alkohol beziehungsweise das Phenol ohne weitere Reinigungsmaßnahmen rückzuführen. Hierzu ist es nicht erforderlich, den Einsatzstoff in hoher Reinheit rückzugewinnen, so daß auch auf eine roh destillierte Fraktion zurückgegriffen werden kann. In diesem Fall sollte die rückzuführende Fraktion weitgehend frei von höher siedendenden Nebenprodukte sein, um die Bildung höhermolekularer Nebenprodukte und Rückstände zu vermindern. Da beim erfindungsgemäßen Verfahren ein hoher Umsatz an Alkohol oder Phenol erzielt wird, kann eine Abtrennung und Rückführung des Ausgangsstoffes in der Regel unterbleiben.

Beim erfindungsgemäßen Verfahren ist es möglich und zumeist vorteilhaft, den Cokatalysator rückzugewinnen und erneut als Cokatalysator einzusetzen, d.h. rückzuführen. Es ist nicht erforderlich, die Cokatalysatoren in hoher Reinheit rückzugewinnen, so daß auch auf eine roh destillierte Fraktion zurückgegriffen werden kann. Allerdings ist es vorteilhaft, die deutlich höher siedenden Produkte abzutrennen. Die gegebenenfalls auftretenden Verluste an Cokatalysatoren sind durch Zugabe frischer Cokatalysatoren zu ersetzen.

Das erfindungsgemäße Verfahren wird besonders bevorzugt eingesetzt zur Herstellung von Alkyl-vinylethern, insbesondere zur Herstellung von tert.-Butyl-vinyl-ethern und Isoamyl-vinylethern.

In einer allgemeinen Ausführungsform wird die basische Alkalimetallverbindung (Katalysator) und der Cokatalysator portionsweise in den flüssigen, gegebenenfalls mit Lösungsmittel verdünnten Alkohol gegeben und durchmischt. Beim Einsatz von Phenolen ist die Verwendung von Lösungsmitteln vorteilhaft. Die entstandene Lösung wird sodann über ein zeolithisches Trocknungsmittel geleitet und einem Rührkessel zugeführt. Durch die Gegenwart des Trocknungsmittels wird das Reaktionswasser entfernt. In die nun nahezu wasserfreie Lösung wird bei einer Temperatur von 100 und 200°C unter intensiver Durchmischung das Acetylen eingeleitet. Beim bevorzugten Einsatz von Ethin wird bevorzugt bis zu einem Druck von 2,4 MPa (24 bar) eingeleitet. Verbrauchtes Acetylen wird nachgeführt. Nach Beendigung der Acetylenaufnahme wird das Reaktionssystem entspannt. Die Reaktionslösung wird in eine Destillationskolonne überführt und der Alkenyl-ether nach Entfernung der leichter siedenden Komponenten über Kopf in hoher Reinheit isoliert.

In einer weiteren allgemeinen Ausführungsform zur Alkenylierung von Alkoholen wird in einem Mischbehälter eine nahezu konzentrierte Lösung (d.h. etwa 80% der maximalen Löslichkeit) der basischen Alkalimetallverbindung im Alkohol hergestellt. Diese Lösung wird kontinuierlich einer Vakuum-Destillationskolonne zugeführt und das gebildete Reaktionswasser über Kopf abgezogen. Die erhaltene wasserfreie Lösung wird kontinuierlich aus dem Sumpf entnommen und mit weiterem, wasserfreiem Alkohol und mit wasserfreiem Cokatalysator versetzt. An dieser Stelle erfolgt auch die Einspeisung der Rückführströme. Die Eduktmischung wird nun einem kontinuierlich arbeitenden Schlaufenreaktor zugeführt. Dort erfolgt die Umsetzung mit dem Acetylen bei einer Temperatur von 100 bis 200°C. Beim bevorzugten Einsatz von Ethin wird bevorzugt bis zu einem Druck von 2,4 MPa (24 bar) eingeleitet. Die Reaktionslösung wird kontinuierlich dem Schlaufenreaktor entnommen und destillativ aufgearbeitet. Der Alkenyl-ether wird als Reinprodukt isoliert. Zurückgewonnener, nicht-umgesetzter Alkohol und der abgetrennte Cokatalysator werden rückgeführt.

In einer dritten, besonders bevorzugten Ausführungsform wird in einem Mischbehälter eine Lösung von 0,05 bis 8 Mol-% Kaliumhydroxid in einem Alkohol hergestellt und mit 0,1 bis 10 Mol-% 1,4-Diethoxy-butan und/oder 1,4-Divinyloxy-butan, bezogen auf die Molmenge des eingesetzten Alkohols, versetzt. Diese Lösung wird kontinuierlich einer Vakuum-Destillationskolonne zugeführt und das gebildete Reaktionswasser über Kopf abgezogen. Die nahezu wasserfreie Lösung wird kontinuierlich aus dem Sumpf entnommen und einem Rührkessel zugeführt. Dort erfolgt die halbkontinuierliche Umsetzung mit dem gasförmigen Ethin bei einer Temperatur von 130 bis 180°C und einem Druck von 0,1 bis 2,0 MPa (1 bis 20 bar). Nach Beendigung der Reaktion wird der Reaktorinhalt ausgeschleust und der destillativen Aufarbeitung zugeführt. Der Alkyl-vinyl-ether wird in hoher Reinheit gewonnen. Die Fraktion, welche den zugesetzten Cokatalysator enthält, kann gegebenenfalls rückgeführt und erneut eingesetzt werden.

Das erfindungsgemäße Verfahren ermöglicht die einfache Herstellung von Alkenyl-ethern durch Umsetzung der entsprechenden Alkohole oder Phenole mit Acetylenen in Gegenwart basischer Alkalimetallverbindungen und eines Cokatalysators mit einer hohen Raum/Zeit-Ausbeute. Insbesondere wird die Bildung von Nebenprodukten bei gleichzeitig konzentrierter Fahrweise erheblich zurückgedrängt, so daß höchstens nur eine geringe Menge an nicht-flüchtigen Rückstand gebildet wird und ein Zähflüssigwerden oder eine Verfestigung des Reaktionsaustrags unterbunden wird. Aufgrund des hohen Umsatzes an Alkohol oder Phenol deutlich über 90%, vielfach über 95%, kann eine Abtrennung und Rückführung des Ausgangsstoffes in der Regel unterbleiben. Das erfindungsgemäße Verfahren ermöglicht auch die Alkenylierung tertiärer Alkohole mit hoher Ausbeute.

Gegenüber den bekannten Verfahren zeichnet sich das erfindungsgemäße Verfahren durch eine konzentrierte Fahrweise aus, was neben den erwähnten Vorteil einer hohen Raum/Zeit-Ausbeute zu einer Verringerung des erforderlichen Reaktionsvolumens und damit verbunden auch zu einer Verringerung der benötigten Energien führt. Im Gegensatz zu den vorbeschriebenen Lösungsmitteln, welche vielfach noch freie Hydroxygruppen besitzen und somit einen erheblichen Teil des zugegebenen Acetylens verbrauchen, zeigen die erfindungsgemäß einzusetzenden Cokatalysatoren keine oder höchstens nur geringe Aufnahme an Acetylen.

Gegenüber den bekannten Verfahren ohne Einsatz eines Lösungsmittels und Cokatalysators zeichnet sich das erfindungsgemäße Verfahren durch eine erhebliche geringere Bildung von Nebenprodukten aus. Geringe Konzentrationen an Cokatalysatoren von weniger als einem Gew.-% wirken beim erfindungsgemäßen Verfahren bereits sehr effektiv.

Die Alkenyl-ether können beim erfindungsgemäßen Verfahren in hoher Reinheit gewonnen werden. Nach Feindestillation sind Reinheiten von größer 99,9% erreichbar. Da auch die eingesetzten Cokatalysatoren in der Regel destillativ abgetrennt werden können, ist deren Rückführung und erneuter Einsatz möglich.

Beispiele

Definitionen

Die in der Beschreibung und den Beispielen angegebenen Werte für Umsatz, Selektivität und Ausbeute sind durch folgende Gleichungen definiert:

$$\text{Umsatz} = [m_{vor}(\text{R-OH}) - m_{nach}(\text{R-OH})] / m_{vor}(\text{R-OH})$$

$$\text{Selektivität} = m_{nach}(\text{Alkenyl-ether}) / [m_{vor}(\text{R-OH}) - m_{nach}(\text{R-OH})]$$

$$\text{Ausbeute} = \text{Umsatz} \times \text{Selektivität} = m_{nach}(\text{Alkenyl-ether}) / m_{vor}(\text{R-OH}).$$

Die für die Berechnung zugrunde gelegten Massen:

$m_{vor}(\text{R-OH})$ eingesetzte Masse an Alkohol oder Phenol

$m_{nach}(\text{R-OH})$ nicht-umgesetzte Masse an Alkohol oder Phenol

$m_{nach}(\text{Alkenyl-ether})$ Masse an gebildetem Alkenyl-ether nach Reindestillation

wurden aus den GC-Flächen-% ermittelt.

Versuchsdurchführung für die Beispiele 1 bis 3

Es wurden jeweils 100 ml tert.-Butanol mit 8 Gew.-% Kalium-tert.butylat versetzt, unter Rühren gelöst und gegebenenfalls 2,5 Gew.-% des Cokatalysators zugegeben. Der Reaktionsansatz wurde in einen 300 ml-Autoklaven gefüllt und bei Raumtemperatur mit Stickstoff auf 0,5 MPa abs (5 bar abs) aufgepresst. Nach Erwärmung auf 160°C wurde mit Ethin auf 2,0 MPa abs (20 bar abs) nachgepresst. Durch Reaktion verbrauchtes Ethin wurde durch kontinuierliches Nachpressen bei 2,0 MPa abs (20 bar abs) nachgeliefert. Nach 12 Stunden wurde der Versuch abgebrochen und das Reaktionsprodukt aufdestilliert. Die Analyse erfolgte gaschromatografisch.

Beispiel 1 (Vergleichsbeispiel ohne Cokatalysator)

Beispiel 1 wurde ohne Zusatz eines Cokatalysators durchgeführt. Der Umsatz an tert.-Butanol betrug 75,9%. Das Wertprodukt tert.-Butyl-vinyl-ether wurde mit einer Ausbeute von 68,4% erhalten. Bei der destillativen Aufarbeitung wurde ein nicht-flüchtiger Rückstand von 6,4 Gew.-%, bezogen auf den Autoklavenaustrag erhalten.

Beispiel 2 (erfindungsgemäß)

In Beispiel 2 wurde 2,5 Gew.-% 1,4-Diethoxy-butan als Cokatalysators zugegeben. Der Umsatz an tert.-Butanol betrug 97,9%. Das Wertprodukt tert.-Butyl-vinyl-ether wurde mit einer Ausbeute von 90,9% erhalten. Bei der destillativen Aufarbeitung wurde ein nicht-flüchtiger Rückstand von 2,7 Gew.-%, bezogen auf den Autoklavenaustrag erhalten.

Beispiel 3 (erfindungsgemäß)

In Beispiel 3 wurde 2,5 Gew.-% 1,4-Divinyloxy-butan als Cokatalysators zugegeben. Der Umsatz an tert.-Butanol betrug 95,4%. Das Wertprodukt tert.-Butyl-vinyl-ether wurde mit einer Ausbeute von 89,1% erhalten. Bei der destillativen Aufarbeitung wurde ein nicht-flüchtiger Rückstand von 2,2 Gew.-%, bezogen auf den Autoklavenaustrag erhalten. Tert.-Butyl-vinyl-ether wurde in einer Reinheit von 99,2 GC-Flächen-% gewonnen.

Beispiel 4 (erfindungsgemäß)

In Beispiel 4 wurden in einen 300 ml-Autoklaven unter Rühren 122,8 g Phenol in 129,1 g N-Methylpyrrolidon gelöst und mit 8 Gew.-% Kaliumhydroxid sowie 2,5 Gew.-% 1,4-Diethoxy-butan versetzt. Der Wassergehalt der Lösung wurde zu 0,74 GC-Flächen-% bestimmt. Nun wurde bei Raumtemperatur mit Stickstoff auf 0,5 MPa abs (5 bar abs) aufgepresst. Nach Erwärmung auf 190°C wurde mit Ethin auf 2,0 MPa abs (20 bar abs) nachgepresst. Durch Reaktion verbrauchtes Ethin wurde durch kontinuierliches Nachpressen bei 2,0 MPa abs (20 bar abs) nachgeliefert. Nach 24 Stunden wurde der Versuch abgebrochen. Die Analyse erfolgte gaschromatografisch. Der Umsatz an Phenol betrug 97,7%. Das Wertprodukt Phenyl-vinylether wurde mit einer Ausbeute von 80,5% erhalten. Bei der destillativen Aufarbeitung wurde ein nicht-flüchtiger Rückstand von 19,9 Gew.-%, bezogen auf den Autoklavenaustrag erhalten.

Eine Zusammenfassung der Beispiele ist in Tabelle 1 gegeben. Unter sonst identischen Bedingungen wurden bei der Vinylierung von tert.-Butanol ohne Cokatalysator die mit Abstand niedrigste Ausbeute von 68,4% erzielt. Die beiden Beispiele mit Cokatalysator zeigen eine deutlich höhere Ausbeute von 89,1 und 90,9%. Durch den positiven Einfluß der Cokatalysatoren wurde deutlich weniger nicht-flüchtiger Rückstand gebildet. Aufgrund des deutlich höheren Umsatzes und der gestiegenen Selektivität wurde in Gegenwart der Cokatalysatoren auch eine deutlich höhere Raum/Zeit-Ausbeute erzielt.

Tabelle 1:

| Nr. | Einsatzstoff | Cokatalysator | Umsatz [%] | Selektivität [%] | Ausbeute [%] | nicht–flüchtiger Rückstand [Gew.–%] |
|---|---|---|---|---|---|---|
| 1 | tert.–Butanol | ohne (Vergleichsbeispiel) | 75,9 | 90,1 | 68,4 | 6,4 |
| 2 | tert.–Butanol | 2,5 Gew.–% 1,4–Diethoxy–butan | 97,9 | 92,8 | 90,9 | 2,7 |
| 3 | tert.–Butanol | 2,5 Gew.–% 1,4–Divinyloxy–butan | 95,4 | 93,4 | 89,1 | 2,2 |
| 4 | Phenol in N–Methylpyrrolidon | 2,5 Gew.–% 1,4–Diethoxy–butan | 97,7 | 82,4 | 80,5 | 19,9 |

EP 1 268 380 B1

**Patentansprüche**

1.  Verfahren zur Herstellung von Alkenyl-ethern durch Umsetzung der entsprechenden Alkohole, ausgewählt aus der Gruppe Methanol, Ethanol, 1-Propanol, 2-Propanol, 1-Butanol, 2-Butanol, 2-Methyl-1-propanol, 1-Methyl-2-propanol, 1-Pentanol, 2-Pentanol, 3-Pentanol, 2-Methyl-1-butanol, 3-Methyl-1-butanol, 2,2-Dimethyl-1-propanol, 2-Methyl-2-butanol, 3- Methyl-2-butanol, 1-Hexanol, 2-Hexanol, 3-Hexanol, 3,3-Dimethyl-3-butanol, 4-Methyl-2-pentanol, 2-Ethyl-1-butanol, cis-3-Hexen-1-ol, 5-Hexen-1-ol, 1-Heptanol, 2-Heptanol, 3-Heptanol, 2,4-Dimethyl-3-pentanol, 1-Octanol, 2-Octanol, 3-Octanol, 2-Ethyl-1-hexanol, 2,4,4-Trimethyl-1-pentanol, 1-Nonanol, 2-Nonanol, 3-Nonanol, 4-Nonanol, 5-Nonanol, 1-Decanol, 2,2-Dimethyl-1-octanol, 1-Dodecanol, 1-Tetradecanol, 1-Hexadecanol, 1-Octadecanol, cis-9-Octadecen-1-ol, cis,cis-9,12-Octadecadien-1-ol, cis,cis,cis- 9,12,15-Octadecatrien-1-ol, 1-Eicosanol, 1-Docosanol, Cyclopropanol, Cyclopropylmethanol, Cyclopropylethanol, Cyclobutanol, Cyclobutylmethanol, Cyclobutylethanol, Cyclopentanol, Cyclopentylmethanol, Cyclopentylethanol, 1-Methyl-cyclopentanol, 2-Methyl-cyclopentanol, 3-Methyl-cyclopentanol, Cyclohexanol, Cyclohexylmethanol, Cyclohexylethanol, 1-Methyl-cyclohexanol, 2-Methyl-cyclohexanol, 3-Methyl-cyclohexanol, 4-Methyl-cyclohexanol, Cycloheptanol, Cyclooctanol, Cyclodecanol, Benzylalkohol, Hydroxydiphenylmethan, 1-Phenyl-ethanol, 2-Phenyl-ethanol, 2,2-Diphenyl-ethanol, 2,2,2-Triphenylethanol, 1,2-Ethandiol, 1,2-Propandiol, 1,3-Propandiol, 1,2-Butandiol, 1,5-Pentandiol, 1,6-Hexandiol, Diethylenglykol, Triethylenglykol, Tetraethylenglykol, 1,2,3-Propantriol, 2-Methyl-1,2,3-propantriol,
    oder der entsprechenden Phenole
    mit Acetylenen in der Flüssigphase in Gegenwart basischer Alkalimetallverbindungen und eines Cokatalysators, **dadurch gekennzeichnet, daß** man als Cokatalysator Verbindungen der allgemeinen Formel (Ia) und/oder (Ib)

    $$\text{(Ia):} \qquad R^1O\text{-}(CH_2CH_2CH_2OH_2O)_n\text{-}H$$

    $$\text{(Ib):} \qquad R^1O\text{-}(CH_2CH_2CH_2CH_2O)_n\text{-}R^2,$$

    worin $R^1$, $R^2$ unabhängig voneinander $C_1$- bis $C_6$-Alkyl oder $C_2$ bis $C_6$-Alkenyl, oder $R^1$ und $R^2$ gemeinsam eine Butenyl-Einheit, bedeuten und n 1, 2, 3, 4 oder 5 entspricht, einsetzt.

2.  Verfahren nach Anspruch 1, **dadurch gekennzeichnet, daß** man als Cokatalysator Verbindungen gemäß den Formeln (1a) und/oder (1b), worin $R^1$, $R^2$ unabhängig voneinander Ethyl oder Vinyl bedeuten, einsetzt.

3.  Verfahren nach den Ansprüchen 1 bis 2, **dadurch gekennzeichnet, daß** man als Cokatalysatoren, 1,4-Diethoxy-butan, 1,4-Divinyloxy-butan oder Gemische davon einsetzt.

4.  Verfahren nach den Ansprüchen 1 bis 3, **dadurch gekennzeichnet, daß** man den Cokatalysatoren (Ia) und/oder (Ib) in einer Menge von 0,1 bis 10 Gew.-%, bezogen auf den eingesetzten Alkohol oder das eingesetzte Phenol, verwendet.

5.  Verfahren nach den Ansprüchen 1 bis 4, **dadurch gekennzeichnet, daß** man die basischen Alkalimetallverbindungen in einer Menge von 0,05 bis 10 % der Molmenge des eingesetzten Alkohols oder Phenols einsetzt.

6.  Verfahren nach den Ansprüchen 1 bis 5, **dadurch gekennzeichnet, daß** man die Umsetzung zwischen den Alkoholen oder Phenolen und den Acetylenen bei einer Temperatur von 100 bis 200°C und einem Acetylendruck von kleiner 5 MPa durchführt.

7.  Verfahren nach den Ansprüchen 1 bis 6, **dadurch gekennzeichnet, daß** man den Cokatalysator rückgewinnt und erneut als Cokatalysator einsetzt.

8.  Verfahren nach den Ansprüchen 1 bis 8, **dadurch gekennzeichnet, daß** man aliphatische Alkohole einsetzt.

9.  Verfahren nach den Ansprüchen 1 bis 8, **dadurch gekennzeichnet, daß** man Alkyl-vinylether herstellt.

## Claims

1. A process for preparing alkenyl ethers by reacting the corresponding alcohols selected from the group consisting of methanol, ethanol, 1-propanol, 2-propanol, 1-butanol, 2-butanol, 2-methyl-1-propanol, 1-methyl-2-propanol, 1-pentanol, 2-pentanol, 3-pentanol, 2-methyl-1-butanol, 3-methyl-1-butanol, 2,2-dimethyl-1-propanol, 2-methyl-2-butanol, 3-methyl-2-butanol, 1-hexanol, 2-hexanol, 3-hexanol, 3,3-dimethyl-3-butanol, 4-methyl-2-pentanol, 2-ethyl-1-butanol, cis-3-hexen-1-ol, 5-hexen-1-ol, 1-heptanol, 2-heptanol, 3-heptanol, 2,4-dimethyl-3-pentanol, 1-octanol, 2-octanol, 3-octanol, 2-ethyl-1-hexanol, 2,4,4-trimethyl-1-pentanol, 1-nonanol, 2-nonanol, 3-nonanol, 4-nonanol, 5-nonanol, 1-decanol, 2,2-dimethyl-1-octanol, 1-dodecanol, 1-tetradecanol, 1-hexadecanol, 1-octadecanol, cis-9-octadecen-1-ol, cis,cis-9,12-octadecadien-1-ol, cis,cis,cis-9,12,15-octadecatrien-1-ol, 1-eicosanol, 1-docosanol, cyclopropanol, cyclopropylmethanol, cyclopropylethanol, cyclobutanol, cyclobutylmethanol, cyclobutylethanol, cyclopentanol, cyclopentylmethanol, cyclopentylethanol, 1-methyl-cyclopentanol, 2-methyl-cyclopentanol, 3-methyl-cyclopentanol, cyclohexanol, cyclohexylmethanol, cyclohexylethanol, 1-methyl-cyclohexanol, 2-methyl-cyclohexanol, 3-methyl-cyclohexanol, 4-methyl-cyclohexanol, cycloheptanol, cyclooctanol, cyclodecanol, benzyl alcohol, hydroxydiphenylmethane, 1-phenylethanol, 2-phenylethanol, 2,2-diphenylethanol, 2,2,2-triphenylethanol, 1,2-ethanediol, 1,2-propanediol, 1,3-propanediol, 1,2-butanediol, 1,5-pentanediol, 1,6-hexanediol, diethylene glycol, triethylene glycol, tetraethylene glycol, 1,2,3-propanetriol and 2-methyl-1,2,3-propanetriol
or the corresponding phenols
with acetylenes in the liquid phase in the presence of basic alkali metal compounds and a cocatalyst comprising compounds of the formula (Ia) and/or (Ib)

$$\text{(Ia):} \qquad R^1O\text{-}(CH_2CH_2CH_2CH_2O)_n\text{-}H$$

$$\text{(Ib):} \qquad R^1O\text{-}(CH_2CH_2CH_2CH_2O)_n\text{-}R^2,$$

where $R^1$, $R^2$ are, independently of one another, $C_1$-$C_6$-alkyl or $C_2$-$C_6$-alkenyl, or $R^1$ and $R^2$ together form a butenyl unit and n is 1, 2, 3, 4 or 5.

2. A process as claimed in claim 1, wherein the cocatalyst used comprises compounds of the formulae (Ia) and/or (Ib), in which $R^1$, $R^2$ are, independently of one another, ethyl or vinyl.

3. A process as claimed in claim 1 or 2, wherein the cocatalyst used is 1,4-diethoxybutane, 1,4-divinyloxybutane or a mixture thereof.

4. A process as claimed in any of claims 1 to 3, wherein the cocatalyst (Ia) and/or (Ib) is used in an amount of from 0.1 to 10% by weight, based on the alcohol used or the phenol used.

5. A process as claimed in any of claims 1 to 4, wherein the basic alkali metal compounds are used in an amount of from 0.05 to 10% of the molar amount of the alcohol or phenol used.

6. A process as claimed in any of claims 1 to 5, wherein the reaction of the alcohols or phenols with the acetylenes is carried out at from 100 to 200°C and an acetylene pressure of less than 5 MPa.

7. A process as claimed in any of claims 1 to 6, wherein the cocatalyst is recovered and reused as cocatalyst.

8. A process as claimed in any of claims 1 to 8, wherein aliphatic alcohols are used.

9. A process as claimed in any of claims 1 to 8, wherein alkyl vinyl ethers are prepared.

## Revendications

1. Procédé de production d'éthers alcényliques au moyen de la réaction des alcools correspondants choisis dans le groupe formé par le méthanol, l'éthanol, le 1-propanol, le 2-propanol, le 1-butanol, le 2-butanol, le 2-méthyl-1-propanol, le 1-méthyl-2-propanol, le 1-pentanol, le 2-pentanol, le 3-pentanol, le 2-méthyl-1-butanol, le 3-méthyl-1-butanol, le 2,2-diméthyl-1-propanol, le 2-méthyl-2-butanol, le 3-méthyl-2-butanol, le 1-hexanol, le 2-hexanol, le 3-hexanol, le 3,3-diméthyl-3-butanol, le 4-méthyl-2-pentanol, le 2-éthyl-1-butanol, le cis-3-hexén-1-ol, le 5-hexén-1-ol, le 1-heptanol, le 2-heptanol, le 3-heptanol, le 2,4-diméthyl-3-pentanol, le 1-octanol, le 2-octanol, le 3-octanol, le 2-éthyl-1-hexanol, le 2,4,4-triméthyl-1-pentanol, le 1-nonanol, le 2-nonanol, le 3-nonanol, le 4-nonanol, le 5-nonanol, le 1-décanol, le 2,2-diméthyl-1-octanol, le 1-dodécanol, le 1-tétradécanol, le 1-hexadécanol, le 1-octadécanol, le cis-9-octadécén-1-ol, le cis-cis-9,12-octadécadién-1-ol, le cis,cis,cis-9,12,15-octadécatrién-1-ol, le 1-eicosanol, le 1-docosanol, le cyclopropanol, le cyclopropylméthanol, le cyclopropyléthanol, le cyclobutanol, le cyclobutylméthanol, le cyclobutyléthanol, le cyclopentanol, le cyclopentylméthanol, le cyclopentyléthanol, le 1-méthyl-cyclopentanol, le 2-méthyl-cyclopentanol, le 3-méthyl-cyclopentanol, le cyclohexanol, le cyclohexylméthanol, le cyclohexyléthanol, le 1-méthyl-cyclohexanol, le 2-méthyl-cyclohexanol, le 3-méthyl-cyclohexanol, le 4-méthyl-cyclohexanol, le cycloheptanol, le cyclooctanol, le cyclodécanol, l'alcool benzylique, l'hydroxydiphénylméthane, le 1-phényl-éthanol, le 2-phényl-éthanol, le 2,2-diphényl-éthanol, le 2,2,2-triphényléthanol, le 1,2-éthanediol, le 1,2-propanediol, le 1,3-propanediol, le 1,2-butanediol, le 1,5-pentanediol, le 1,6-hexanediol, le diéthylèneglycol, le triéthylèneglycol, le tétraéthylèneglycol, le 1,2,3-propanetriol, le 2-méthyl-1,2,3-propanetriol, ou des phénols correspondants

avec des acétylènes en phase liquide, en présence de composés de métaux alcalins basiques et d'un co-catalyseur, **caractérisé en ce que**, en tant que co-catalyseur, des composés de formule générale (Ia) et/ou (Ib) sont utilisés

(Ia):     $R^1O\text{-}(CH_2CH_2CH_2CH_2O)_n\text{-}H$

(Ib):     $R^1O\text{-}(CH_2CH_2CH_2CH_2O)_n\text{-}R^2$,

dans lesquelles $R^1$, $R^2$ représentent, indépendamment l'un de l'autre, un groupe alkyle en $C_1$ à $C_6$ ou alcényle en $C_2$ à $C_6$, ou bien $R^1$ et $R^2$ représentent ensemble un motif butényle, et n vaut 1, 2, 3, 4 ou 5.

2. Procédé selon la revendication 1, **caractérisé en ce que**, en tant que co-catalyseur, des composés de formule (Ia) et/ou (Ib) sont utilisés, dans lesquelles $R^1$, $R^2$ représentent, indépendamment l'un de l'autre, des groupes éthyle ou vinyle.

3. Procédé selon les revendications 1 à 2, **caractérisé en ce que**, en tant que co-catalyseurs, le 1,4-diéthoxy-butane, le 1,4-divinyloxy-butane ou des mélanges de ceux-ci sont utilisés.

4. Procédé selon les revendications 1 à 3, **caractérisé en ce que** les co-catalyseurs (Ia) et/ou (Ib) sont utilisés à raison de 0,01% à 10% en poids par rapport à l'alcool utilisé ou au phénol utilisé.

5. Procédé selon les revendications 1 à 4, **caractérisé en ce que** les composés de métaux alcalins basiques sont utilisés à raison de 0,05% à 10% en poids par rapport à la quantité molaire de l'alcool ou du phénol utilisé.

6. Procédé selon les revendications 1 à 5, **caractérisé en ce que** la réaction entre les alcools ou les phénols et les acétylènes est réalisée à une température allant de 100°C à 200°C et sous une pression d'acétylène inférieure à 5 MPa.

7. Procédé selon les revendications 1 à 6, **caractérisé en ce que** le co-catalyseur est récupéré et réutilisé en tant que co-catalyseur.

8. Procédé selon les revendications 1 à 7, **caractérisé en ce que** des alcools aliphatiques sont utilisés.

9. Procédé selon les revendications 1 à 8, **caractérisé en ce que** des éthers alkyl-vinyliques sont produits.